# EUROPEAN PATENT SPECIFICATION

(11) **EP 1 716 828 B1**
(45) Date of publication and mention of the grant of the patent: **21.05.2008**
(21) Application number: 06111987.1
(22) Date of filing: 30.03.2006
(51) Int. Cl.: A61F 9/007, A61M 1/00

(54) **Low resistance irrigation system and apparatus**
Spülsystem mit geringem Widerstand
Système d'irrigation à faible résistance

(30) Priority: 26.04.2005 US 114514
(43) Date of publication of application: 02.11.2006
(73) Proprietor: Alcon, Inc., 6331 Hünenberg (CH)
(72) Inventor: Liao, Grace, Irvine CA California CA 92620 (US); Brody, George, San Clemente CA California CA 92672 (US); Oliveira, Mel M., Huntington Beach CA California CA 92649 (US)
(74) Representative: Hanna, Peter William Derek

(56) References cited:
- US-A- 5 549 547
- US-A1- 2003 195 460

## Description

### Background of the Invention

This invention relates generally to the field of cataract surgery and more particularly to a control system for a phacoemulsification handpiece.

The human eye in its simplest terms functions to provide vision by transmitting light through a clear outer portion called the cornea, and focusing the image by way of the lens onto the retina. The quality of the focused image depends on many factors including the size and shape of the eye, and the transparency of the cornea and lens.

When age or disease causes the lens to become less transparent, vision deteriorates because of the diminished light which can be transmitted to the retina. This deficiency in the lens of the eye is medically known as a cataract. An accepted treatment for this condition is surgical removal of the lens and replacement of the lens function by an artificial intraocular lens (IOL).

In the United States, the majority of cataractous lenses are removed by a surgical technique called phacoemulsification. During this procedure, a thin phacoemulsification cutting tip is inserted into the diseased lens and vibrated ultrasonically. The vibrating cutting tip liquifies or emulsifies the lens so that the lens may be aspirated out of the eye. The diseased lens, once removed, is replaced by an artificial lens.

A typical ultrasonic surgical device suitable for ophthalmic procedures consists of an ultrasonically driven handpiece, an attached cutting tip, and irrigating sleeve and an electronic control console. The handpiece assembly is attached to the control console by an electric cable and flexible fluid tubings. Through the electric cable, the console varies the power level transmitted by the handpiece to the attached cutting tip and the flexible fluid tubings supply irrigation fluid to and draw aspiration fluid from the eye through the handpiece assembly.

The operative part of the handpiece is a centrally located, hollow resonating bar or horn directly attached to a set of piezoelectric crystals. The crystals supply the required ultrasonic vibration needed to drive both the horn and the attached cutting tip during phacoemulsification and are controlled by the console. The crystal/horn assembly is suspended within the hollow body or shell of the handpiece by flexible mountings. The handpiece body terminates in a reduced diameter portion or nosecone at the body's distal end. The nosecone is externally threaded to accept the irrigation sleeve. Likewise, the horn bore is internally threaded at its distal end to receive the external threads of the cutting tip. The irrigation sleeve also has an internally threaded bore that is screwed onto the external threads of the nosecone. The cutting tip is adjusted so that the tip projects only a predetermined amount past the open end of the irrigating sleeve. Ultrasonic handpieces and cutting tips are more fully described in U.S. Pat. Nos. 3,589,363; 4,223,676; 4,246,902; 4,493,694; 4,515,583; 4,589,415; 4,609,368; 4,869,715; 4,922,902; 4,989,583; 5,154,694 and 5,359,996.

In use, the ends of the cutting tip and irrigating sleeve are inserted into a small incision of predetermined width in the cornea, sclera, or other location. The cutting tip is ultrasonically vibrated along its longitudinal axis within the irrigating sleeve by the crystal-driven ultrasonic horn, thereby emulsifying the selected tissue in situ. The hollow bore of the cutting tip communicates with the bore in the horn that in turn communicates with the aspiration line from the handpiece to the console. A reduced pressure or vacuum source in the console draws or aspirates the emulsified tissue from the eye through the open end of the cutting tip, the cutting tip and horn bores and the aspiration line and into a collection device. The aspiration of emulsified tissue is aided by a saline flushing solution or irrigant that is injected into the surgical site through the small annular gap between the inside surface of the irrigating sleeve and the cutting tip.

The preferred surgical technique is to make the incision into the anterior chamber of the eye as small as possible in order to reduce the risk of induced astigmatism. These small incisions result in very tight wounds that squeeze the irrigating sleeve. Such a tight wound construction decreases the stability of the eye, particularly when high aspiration vacuums (above 500 mm Hg) and/or high flows (in excess of 40 cc/min.) are used, because changes in the irrigation flow caused by either changes in the aspiration flow rate or by rapid changes in aspiration vacuum cannot be damped by the inflow of irrigation fluid, which is restricted. Theoretically, increasing the amount of irrigating fluid entering the eye will help to stabilize the intraocular pressure ("IOP"); however, in a clinical setting, the amount of irrigation fluid entering the eye is limited to the amount of fluid aspirated from the eye due to the tight wound construction with minimal leakage from the wound. Also, increasing the flow of irrigating fluid through the eye increases the turbulence in the eye, possibly leading to endothelial cell loss, postoperative inflammation and edema.

US-5,549,547 (Symbiosis Corporation) describes an endoscopic irrigation instrument having a flexible tubing connecting a fluid chamber to a handpiece at its distal end, the distal end of the tubing being tapered and having a smaller internal diameter than the proximal end. The preferred proximal inner diameter is 0.185 inches (4.70 mm), and preferred distal inner diameter is 0.130 inches (3.30 mm). The preferred proximal outer diameter is 0.269 inches (6.84 mm), and preferred distal outer diameter is 0.178 inches (4.52 mm).

Therefore, a need continues to exist for a system that helps to maintain a stable IOP even at high aspiration vacuum levels.

### Brief Summary of the Invention

The present invention improves upon the prior art by providing a surgical irrigation system having reduced irrigation flow resistance, in accordance with claims which follow. Reduction in irrigation fluid flow resistance is achieved by increasing the diameter of the irrigation fluid tubings. The ends of the tubings are tapered to reduce the stiffness of the tubings and to allow the tubings to be connected to current surgical devices.

Accordingly, one objective of the present invention is to provide a surgical irrigation system having reduced irrigation flow resistance.

Another objective of the present invention is to provide a surgical irrigation system having more stable intraocular pressures.

Another objective of the present invention is to provide a surgical irrigation system that allows for higher aspiration vacuum.

Another objective of the present invention is to provide a surgical irrigation system that allows for higher aspiration flow.

These and other advantages and objectives of the present invention will become apparent from the detailed description and claims that follow.

### Brief Description of the Drawings

FIG. 1 is a perspective view of a handpiece and control console that may be used with the present invention.
FIG. 2 is a schematic representation of the handpiece and control console illustrated in FIG. 1.
FIG. 3 is a transverse cross-sectional view of the cutting tip of the present invention taken at line 3-3 in FIG. 2.

### Detailed Description of the Invention

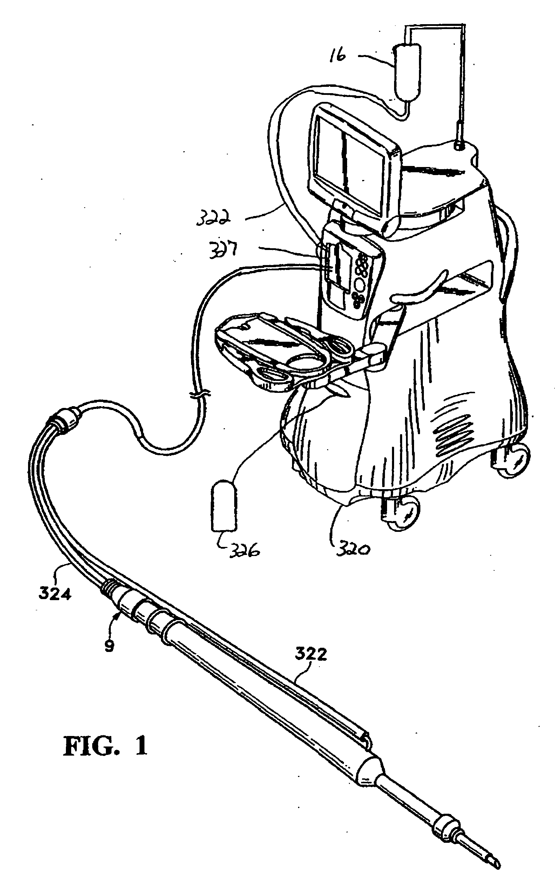

As best seen in FIG. 1, surgical console 320 suitable for use with the present invention may be any commercially available surgical control console such as the INFINITI® surgical systems available from Alcon Laboratories, Inc., Fort Worth, Texas. Console 320 is connected to handpiece 9 through irrigation line 322 and aspiration line 324, and the flow through lines 322 and 324 is controlled by the user, for example, via footswitch 326.

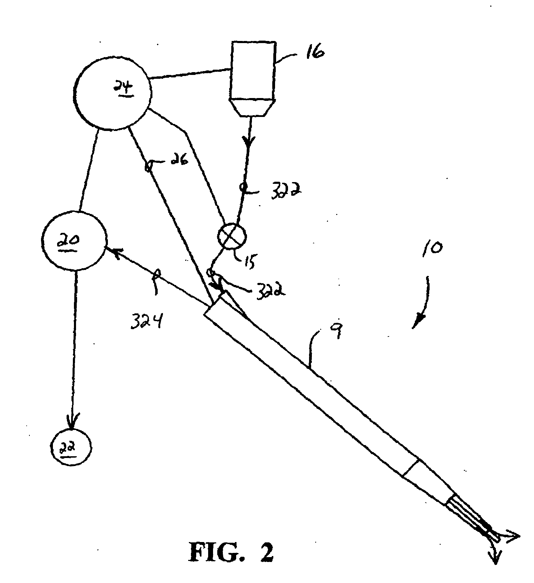

As seen in FIG. 2, schematically, system 10 embodied in console 320 that may be used in the present invention generally included handpiece 9, which is supplied with irrigating fluid through tubings 322 from source 16. Tubings 322 may contain check valve 15 or some other suitable device for controlling the flow of irrigating fluid in tubings 322. The infusion fluid from source 16 is pressurized either by gravity or by pressurizing source 16. Aspiration line 324 fluidly connects handpiece 12 to pump 20, which aspiration fluid for a surgical site and empties the aspirated fluid into container 22. Handpiece 9 is also electronically connected to control module 24 by cable 26. Control module 24 is contained within console 320 and operates to control aspiration pump 20, infusion source 16, valve 15 and the power supplied to handpiece 12.

Change in the intraocular pressure is directly proportional to the irrigation fluid flow resistance in the irrigation system. Therefore, by reducing the irrigation fluid flow resistance in the irrigation system, a more stable IOP can be maintained, even at high aspiration vacuums, without increased irrigation fluid flow. This reduction in the irrigation fluid flow resistance in the irrigation system is best accomplished by increasing the internal and external of tubings 322. Using irrigation tubings 322 having an internal diameter of 4.83 mm (0.190 inches) and having an external diameter of 7.14 mm (0.281 inches) allows for vastly increased irrigation fluid free flow rates (up to approximately 148 cc/min) indicating greatly reduced resistance to flow in tubings 322. One drawback of using such large diameter tubings is that current fittings used on cassettes, check valves, handpieces, etc., are sized to be used with smaller I.D. and O.D. tubing. Increasing the diameters of tubings 322 requires the fitting on all devices in the fluid pathway to be redesigned and/or resized. In addition, larger diameter tubing is stiffer than smaller diameter tubing. Stiffening tubing 322 where it connects to handpiece 9 makes handpiece 9 more difficult to manipulate, resulting in decreased feel and mobility, which is undesirable.

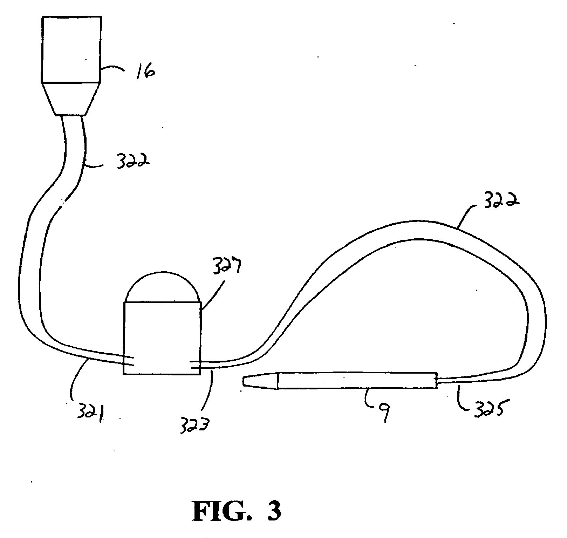

As best seen in FIG. 3, the inventors have discovered a way to increase the diameter of tubing without corresponding decrease in handpiece mobility by reducing the diameter of tubings 322 at portions 321, 323 and 325 where tubings 322 connect to cassette 327 and handpiece 9. Portions 321, 323 and 325 have an internal diameter of 4.06 mm (0.160 inches) and having an external diameter of 4.83 mm (0.190 inches). Such reduction in diameter allows tubings 322 to be more easily connected to conventional cassette 327 and handpiece 9 without modification and decreases the stiffness of portion 325 near handpiece 9. Tubings 322 are of increased interior and exterior diameters along a substantial portion of the length of tubings 322, with reduced diameter portions 321, 323 and 325 making up on a relatively short portion of the length of tubings 322, for example, between approximately 304.8 mm (12.0 inches) and 609.6 mm (24.0 inches).

This description is given for purposes of illustration and explanation. It will be apparent to those skilled in the relevant art that changes and modifications may be made to the invention described above without departing from its scope.

## Claims

1. A surgical system (10), comprising:
a) a surgical console (320);
b) a source (16) of irrigation fluid associated with the surgical console;
c) a phacoemulsification handpiece (9); and
d) a fluid tubing (322) having a distal end and a proximal end, the fluid tubing connected to the handpiece at the distal end and connected to the source of irrigation fluid at the proximal end, wherein the fluid tubing has a first internal diameter at the proximal end, the first internal diameter extending along a substantial portion of a length of the fluid tubing (322), the fluid tubing further having a second internal diameter less than the first internal diameter along a portion (321,323,325) of the fluid tubing at the distal end of the fluid tubing, the portion of the fluid tubing having the second internal diameter being shorter than the substantial portion of the length of the fluid tubing having the first internal diameter,
wherein said first internal diameter is 4.83 mm (0.190 inches);
and said second internal diameter is 4.06 mm (0.16 inches).

2. The surgical system of claim 1, wherein said surgical console comprises a surgical cassette (327), and wherein said fluid tubing (322) comprises intermediate portions (321,323) connectable to said cassette, said intermediate portions having said second internal diameter.

3. The surgical system of claim 1 or claim 2, wherein said fluid tubing has a first external diameter of 7.14 mm (0.281 inches) corresponding with said first internal diameter, and a second external diameter of 4.83 mm (0.19 inches) corresponding to said second internal diameter.

4. A fluid tubing (322) having a distal end and a proximal end, the fluid tubing being adapted to be connected to a phacoemulsification handpiece (9) at the distal end and to a source (16) of irrigation fluid at the proximal end, the fluid tubing having a first internal diameter at the proximal end, the first internal diameter extending along a substantial portion of a length of the fluid tubing and being 4.83 mm (0.190 inches), the fluid tubing further having a second internal diameter being 4.06 mm (0.16 inches) along a portion (321,323,325) of the fluid tubing at the distal end of the fluid tubing, the portion of the fluid tubing having the second internal diameter being shorter than the substantial portion of the length of the fluid tubing having the first internal diameter.

## Patentansprüche

1. Chirurgisches System (10), das Folgendes aufweist:
a) eine chirurgische Konsole (320);
b) eine Quelle (16) für ein Spülfluid, die der chirurgischen Konsole zugeordnet ist;
c) ein Phakoemulsifikationshandstück (9); und
d) einen Fluidschlauch (322) mit einem distalen Ende und einem proximalen Ende, wobei der Fluidschlauch mit dem Handstück an dem distalen Ende verbunden ist und mit der Quelle des Spülfluids an dem proximalen Ende verbunden ist, wobei der Fluidschlauch einen ersten Innendurchmesser an dem proximalen Ende besitzt, der erste Innendurchmesser sich entlang eines wesentlichen Abschnitts einer Länge des Fluidschlauchs (322) erstreckt, der Fluidschlauch weiterhin einen zweiten Innendurchmesser, der geringer als der erste Innendurchmesser ist, entlang eines Abschnitts (321, 323, 325) des Fluidschlauchs an dem distalen Ende des Fluidschlauchs aufweist, der Abschnitt des Fluidschlauchs mit dem zweiten Innendurchmesser kürzer ist als der wesentliche Abschnitt der Länge des Fluidschlauchs mit dem ersten Innendurchmesser, und
wobei der erste Innendurchmesser 4,83 mm (0,190 Zoll) und der zweite Innendurchmesser 4,06 mm (0,16 Zoll) beträgt.

2. Chirurgisches System nach Anspruch 1, bei dem die chirurgische Konsole eine chirurgische Kassette (327) aufweist und bei dem der Fluidschlauch (322) mittlere Abschnitte (321, 323) aufweist, die mit der Kassette verbindbar sind, wobei die mittleren Abschnitte den zweiten Innendurchmesser aufweisen.

3. Chirurgisches System nach Anspruch 1 oder Anspruch 2, bei welchem der Fluidschlauch einen ersten zweiten Außendurchmesser von 7,14 mm (0,281 Zoll) entsprechend dem ersten Innendurchmesser und einen Außendurchmesser von 4,83 mm (0,19 Zoll) entsprechend dem zweiten Innendurchmesser aufweist.

4. Fluidschlauch (322) mit einem distalen Ende und einem proximalen Ende, wobei der Fluidschlauch dafür eingerichtet ist, mit einem Phakoemulsifikationshandstück (9) an dem distalen Ende und mit einer Quelle (16) eines Spülfluids an dem proximalen Ende verbunden zu werden, der Fluidschlauch einen ersten Innendurchmesser an seinem proximalen Ende aufweist, der erste Innendurchmesser sich entlang eines wesentlichen Abschnitts der Länge des Fluidschlauchs erstreckt und 4,83 mm (0,190 Zoll) beträgt, der Fluidschlauch weiterhin einen zweiten Innendurchmesser, der 4,06 mm (0,16 Zoll) entlang eines Abschnitts (321, 323, 325) des Fluidschlauchs an dem distalen Ende des Fluidschlauchs aufweist, und der Abschnitt des Fluidschlauchs mit dem zweiten Innendurchmesser kürzer ist als der wesentliche Abschnitt der Länge des Fluidschlauchs mit dem ersten Innendurchmesser.

## Revendications

1. Système chirurgical (10), comprenant :
a) une console chirurgicale (320),
b) une source (16) de fluide d'irrigation associée à la console chirurgicale,
c) une pièce à main de phacoémulsification (9), et
d) une tubulure de fluide (322) ayant une extrémité distale et une extrémité proximale, la tubulure de fluide étant reliée à la pièce à main à l'extrémité distale, et reliée à la source de fluide d'irrigation à l'extrémité proximale, la tubulure de fluide ayant un premier diamètre interne à l'extrémité proximale, le premier diamètre interne s'étendant le long d'une partie importante de la longueur de la tubulure de fluide (322), la tubulure de fluide ayant en outre un second diamètre intérieur inférieur au premier diamètre interne le long d'une partie (321, 323, 325) de la tubulure de fluide située à l'extrémité distale de la tubulure de fluide, la partie de la tubulure de fluide ayant le second diamètre interne étant plus courte que la partie importante de la longueur de la tubulure de fluide ayant le premier diamètre interne,
dans lequel ledit premier diamètre interne est de 4,83 mm (0,190 pouce), et ledit second diamètre interne est de 4,06 mm (0,16 pouce).

2. Système chirurgical selon la revendication 1, dans lequel ladite console chirurgicale comprend une cassette chirurgicale (327), et dans lequel ladite tubulure de fluide (322) comporte des parties intermédiaires (321, 323) pouvant être reliées à ladite cassette, lesdites parties intermédiaires ayant ledit second diamètre interne.

3. Système chirurgical selon la revendication 1 ou 2, dans lequel ladite tubulure de fluide a un premier diamètre externe de 7,1 mm (0,281 pouce) correspondant audit premier diamètre interne, et un second diamètre externe de 4,83 mm (0,19 pouce) correspondant audit second diamètre interne.

4. Tubulure de fluide (322) ayant une extrémité distale et une extrémité proximale, la tubulure de fluide étant adaptée pour être connectée à une pièce à main de phacoémulsification (9) au niveau de l'extrémité distale, et à une source (16) de fluide d'irrigation au niveau de l'extrémité proximale, la tubulure de fluide ayant un premier diamètre interne à l'extrémité proximale, le premier diamètre interne s'étendant le long d'une partie importante de la longueur de la tubulure de fluide et étant de 4,83 mm (0,190 pouce), la tubulure de fluide ayant en outre un second diamètre interne de 4,06 mm (0,16 pouce) le long d'une partie (321, 322, 325) de la tubulure de fluide située au niveau de l'extrémité distale de la tubulure de fluide, la partie de la tubulure de fluide ayant le second diamètre interne étant plus courte que la partie importante de la longueur de la tubulure de fluide ayant le premier diamètre interne.
